# EUROPÄISCHE PATENTSCHRIFT

(11) **EP 3 145 907 B1**
(45) Veröffentlichungstag und Bekanntmachung des Hinweises auf die Patenterteilung: **27.02.2019**
(21) Anmeldenummer: 15726071.2
(22) Anmeldetag: 22.05.2015
(51) Int. Cl.: C07C 253/30, C07C 255/54

(54) **VERFAHREN ZUR HERSTELLUNG VON ALKOXYBENZONITRILEN**
METHOD FOR PRODUCING ALKOXYBENZONITRILES
PROCÉDÉ DE PRÉPARATION D'ALKOXYBENZONITRILES

(30) Priorität: 23.05.2014 DE 102014007527
(43) Veröffentlichungstag der Anmeldung: 29.03.2017
(73) Patentinhaber: AlzChem Trostberg GmbH, 83308 Trostberg (DE)
(72) Erfinder: TAUBMANN, Christian, 5626 Hermetschwill-Staffeln (CH); KRIMMER, Hans-Peter, 84558 Kirchweidach (DE); HAMBERGER, Josef, 83308 Trostberg (DE); BUB, Joachim, 84478 Waldkraiburg (DE)
(74) Vertreter: Weickmann & Weickmann PartmbB
(86) Internationale Anmeldenummer: PCT/EP2015/061398
(87) Internationale Veröffentlichungsnummer: WO 2015/177344

(56) Entgegenhaltungen:
- WO-A1-03/097584
- CN-A- 101 481 329
- US-A1- 2007 197 532
- OLALLA VÁZQUEZ ET AL: "Bis-4-aminobenzamidines: Versatile, Fluorogenic A/T-Selective dsDNA Binders", ORGANIC LETTERS, Bd. 12, Nr. 2, 15. Januar 2010 (2010-01-15), Seiten 216-219, XP55204419, ISSN: 1523-7060, DOI: 10.1021/ol902501j
- GROMYKO A V ET AL: "DNA Sequence-Specific Ligands: XII. Synthesis and Cytological Studies of Dimeric Hoechst 33258 Molecules", RUSSIAN JOURNAL OF BIOORGANIC CHEMISTRY, KLUWER ACADEMIC PUBLISHERS-PLENUM PUBLISHERS, NE, Bd. 31, Nr. 4, 1. Juli 2005 (2005-07-01), Seiten 344-351, XP019299958, ISSN: 1573-9163
- "CRC Handbook of Chemistry and Physics 76th Edition 1995 - 1996", 1996 pages 159-192,

## Beschreibung

Die vorliegende Erfindung betrifft ein Verfahren zur Herstellung von Alkoxybenzonitrilen, in dem halogenierte Benzonitrile in Gegenwart von Natrium-methylsulfinylmethid mit Alkoholen zur Reaktion gebracht werden.

Halogenierte Benzonitrile stellen industriell bedeutsame, multifunktionale Synthesebausteine dar. Sowohl die Nitrilgruppe(n) als auch die Halogensubstituenten bieten Anknüpfungspunkte für eine reichhaltige Folgechemie. Ein klassisches Beispiel für eine Reaktion unter Erhalt des Halogensubstituenten ist die Umsetzung von p-Chlorbenzonitril mit Bernsteinsäurediestern zum Diketopyrrolopyrrolsystem des Pigments PR 254, das sich durch eine hervorragende chemische Stabilität und Witterungsbeständigkeit auszeichnet. Zielt hingegen die Folgereaktion an sich bzw. zunächst auf eine Funktionalisierung unter Substitution der Chlorfunktion des p-Chlorbenzonitrils und gleichzeitigem Erhalt der Nitrilgruppe ab, so erschwert gerade die Stabilität der Aryl-Chlor-Bindung die Umsetzung. Während die Fluor-, Brom- und lodbenzonitrile gegenüber einer nucleophilen Substitution eine bekanntermaßen höhere Reaktivität aufweisen, sind die wirtschaftlich günstigeren chlorierten Benzonitrile in der Regel deutlich reaktionsträger. Insbesondere für eine C-O-Kupplung sind die Synthesemethoden entweder auf wenige Substrate beschränkt oder aufgrund teurer Reagenzien nicht wirtschaftlich.

So gelingt zwar die Reaktion von nicht aktivierten Halogenbenzonitrilen mit Alkoholen zu den entsprechenden alkoxysubstituierten Systemen unter Verwendung von Alkalialkoholaten (WO 2003/097584 A1). Jedoch sind nur die Alkalialkoholate kurzkettiger, einwertiger Alkohole auf einfache und wirtschaftliche Weise zugänglich und daher großtechnisch verfügbar. Beispiele hierfür sind Lithium-, Natrium- und Kaliumsalze der Alkohole Methanol, Ethanol sowie *tert*-Butanol. Zudem muss für eine effektivere Umsetzung oft auf teure und in der Folge meist schwer abtrennbare Übergangsmetallkatalysatoren zurückgegriffen werden. So beschreibt beispielsweise WO 2008/136770 A1 die C-O-Kupplung mit Hilfe von NHC-Nickel-Katalysatoren. Nickel- sowie Palladiumsysteme werden in J. Org. Chem. 2009, 74, 1971-1976 beschrieben. Kupferkatalysierte Varianten werden in Synlett (2011), (10), 1419-1422 vorgestellt.

Gerade bei der Ausweitung dieser C-O-Kupplungsreaktion auf mittel- oder langkettige und/oder mehrwertige Alkohole sind bislang die Möglichkeiten stark eingeschränkt, da die entsprechenden Alkoholate nur schwer mit zufriedenstellender Ausbeute und in ausreichender Reinheit hergestellt werden können und daher oft kommerziell nicht erhältlich sind. So wird zum Beispiel für Dinatrium-1,6-hexandiolat lediglich ein Verfahren beschrieben, das sich jedoch nicht durchgesetzt hat (DE 968903). Überraschenderweise scheitern Laborversuche zur direkten Darstellung dieses Salzes aus 1,6-Hexandiol und metallischem Natrium, da zum einen die Deprotonierung bei Raumtemperatur scheinbar kinetisch stark gehemmt ist, zum anderen eine erhöhte Reaktionstemperatur zu Nebenreaktionen führt. Werden bei unvollständigem Umsatz Gemische erhalten, lassen sich diese aufgrund der schlechten Löslichkeit der Zielverbindung nur schwer aufreinigen. Insgesamt sind die Einsatzmöglichkeiten von mittel- und langkettigen und/oder mehrwertigen Alkoholen in dieser Art von Reaktion folglich sehr stark eingeschränkt und unbefriedigend.

Wirtschaftlich bedeutende Folgeprodukte, die sich formal von diesen Alkoholen ableiten, werden daher bislang in großem Umfang hergestellt, indem man sich der inversen Reaktivität bedient, das heißt, entsprechende Hydroxybenzonitrile bzw. deren Salze einerseits und Halogenalkane andererseits als Edukte verwendet. Als Beispiele können zahlreiche Zielstrukturen von Kosmetika und Pharmazeutika dienen, insbesondere Vorstufen der Verbindungen Pentamidin und Hexamidin sowie deren Derivate. So wird die Hexamidin-Vorstufe 1,6-*Bis*(4-cyanophenoxy)hexan ausgehend von 4-Hydroxybenzonitril, einem alpha,omega-Hexyldihalogenid sowie einer Base hergestellt, wobei für eine ausreichend hohe Reaktivität meist auf die Bromverbindung 1,6-Dibromhexan zurückgegriffen werden muss (CN 101481329). Dies ist im Vergleich zum Chloranalogon nicht nur mit höheren Rohstoffkosten verbunden, sondern zieht auch mehrere Probleme nach sich, wie zum Beispiel eine höhere Korrosivität der freigesetzten Bromide und höhere Entsorgungskosten für bromhaltigen Abfall. Gleichzeitig kommt aber der Veredelung langkettiger und/oder mehrwertiger Alkohole - insbesondere aus nachwachsenden Rohstoffen - eine immer wichtigere Bedeutung zu. Ein Verfahren, das oben genannte Nachteile unter Nutzung mittel- oder langkettiger und/oder mehrwertiger Alkohole umgeht, wäre daher wünschenswert.

O. Väsquez et al. (Org. Let., Vol. 12, No. 2, 2010, 216-219) betrifft bis-4-Aminobenzamidine. In dem Artikel wird ein Verfahren zur Herstellung von Alkoxybenzonitrilen beschrieben durch Umsetzung von p-Fluor-benzonitril mit Diolen in THF/DMF in Gegenwart von NaH. Das Lösungsmittel Tetrahydrofuran (THF) weist eine Permittivität von 7,52 auf.

A. V. Gromyko et al. (Russian Journal of Bioorganic Chemistry, Vol. 31, No. 4, 2005, 344-351) beschreibt die Herstellung von Alkoxybenzonitrilen ausgehend von Hydroxybenzonitril und di-Halogenalkan bzw. Halogenalkylether unter Verwendung von NaH in DMF.

Aufgabe der vorliegenden Erfindung ist somit, ein Verfahren zur Herstellung von Alkoxybenzonitrilen bereitzustellen, in dem halogenierte Benzonitrile und Alkohole als Ausgangsstoffe umgesetzt werden, und die Nachteile des Standes der Technik zu beheben. Des Weiteren ist es Aufgabe der vorliegenden Erfindung, ein verbessertes Verfahren zur Herstellung von Alkoxybenzonitrilen bereitzustellen, das eine hohe Raum-Zeit-Ausbeute liefert, auf einer günstigen Kostenbasis beruht und großtechnisch einsetzbar ist, als auch ein Verfahren bereitzustellen, mit welchem die gewünschten Produkte in hoher Reinheit bereitgestellt werden können, insbesondere ohne dass ein aufwändiges Aufarbeitungsverfahren nachgeschaltet werden muss.

Gelöst wird diese Aufgabe durch ein Verfahren gemäß Anspruch 1. Demnach ist ein Verfahren zur Herstellung von Alkoxybenzonitrilen gemäß Formel (I) Gegenstand der vorliegenden Erfindung 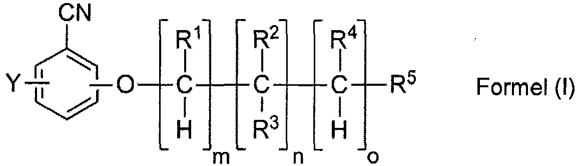 worin für die Reste Y und R⁵ gleichzeitig oder unabhängig voneinander gilt:
- R⁵ =: Wasserstoff, Hydroxy oder ein Rest gemäß Formel (II) mit p = eine ganze Zahl von 0 bis 4 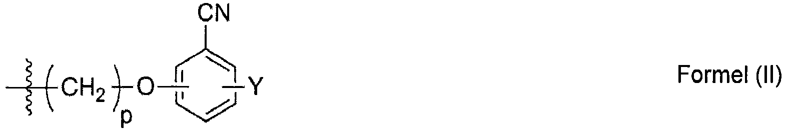
- Y =: jeweils gleichzeitig oder unabhängig voneinander Wasserstoff, Fluor, Chlor, Brom oder Iod;
und worin für die Reste R¹, R², R³, R⁴ und die Indices m, n, o gleichzeitig oder unabhängig voneinander gilt:
a) R¹, R², R³, R⁴ = gleichzeitig oder unabhängig voneinander Wasserstoff, Hydroxy, C1-C6-Alkyl, C1-C6-Hydroxyalkyl oder
   ein Rest gemäß oben angegebener Formel (II) und Bedeutung;
   m = eine ganze Zahl von 1 bis 4;
   n, o = gleichzeitig oder unabhängig voneinander eine ganze Zahl von 0 bis 4;
   oder
b) R¹ und R² = gemeinsam unter Bildung eines Ringes C1-C6-Alkylen, oder C1-C6-Hydroxyalkylen;
   R³, R⁴ = gleichzeitig oder unabhängig voneinander Wasserstoff, Hydroxy, C1-C6-Alkyl, C1-C6-Hydroxyalkyl oder ein Rest gemäß oben angegebener Formel (II) und Bedeutung;
   m, n = gleichzeitig oder unabhängig voneinander eine ganze Zahl von 1 bis 4;
   o = eine ganze Zahl von 0 bis 4;
   oder
c) R¹ und R⁴ = gemeinsam unter Bildung eines Ringes C1-C6-Alkylen, oder C1-C6-Hydroxyalkylen;
   R², R³ = gleichzeitig oder unabhängig voneinander Wasserstoff, Hydroxy, C1-C6-Alkyl, C1-C6-Hydroxyalkyl oder ein Rest gemäß oben angegebener Formel (II) und Bedeutung;
   m, o = gleichzeitig oder unabhängig voneinander eine ganze Zahl von 1 bis 4;
   n = eine ganze Zahl von 0 bis 4;
in dem ein Halogenbenzonitril gemäß Formel (III) mit einem Alkohol gemäß Formel (IV) zur Reaktion gebracht wird 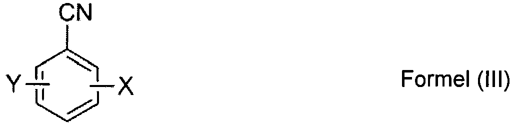 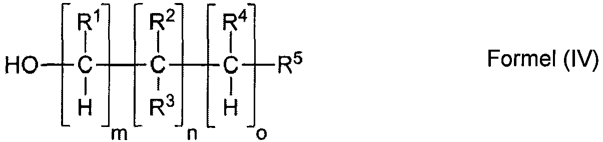 worin die Reste R¹, R², R³, R⁴, R⁵, Y und die Indices m, n, o die oben angegebene Bedeutung aufweisen und für den Rest X gilt:
X = Fluor, Chlor, Brom oder Iod;
wobei die Reaktion in einem von Dimethylsulfoxid verschiedenen, polaren aprotischen Lösungsmittel in Gegenwart von Natrium-methylsulfinylmethid durchgeführt wird, wobei das polare aprotische Lösungsmittel eine Permittivität von über 15 aufweist.

Überraschenderweise stellt sich die gewünschte Reaktivität zwischen mittel- und langkettigen oder/und mehrwertigen Alkoholen mit aktivierten oder/und nichtaktivierten Halogenbenzonitrilen ein, wenn die Reaktion in einem von Dimethylsulfoxid (DMSO; (CH₃)₂SO)) verschiedenen, polaren aprotischen Lösungsmittel in Gegenwart von Natrium-methylsulfinylmethid durchgeführt wird. Hierbei kann die Reaktion als Eintopfreaktion geführt werden, wenn die Deprotonierung des Alkohols in situ erfolgt und ein System aus Natriumhydrid und Dimethylsulfoxid in dem polaren aprotischen Lösungsmittel verwendet wird. Bekanntermaßen bildet sich aus letzterer Kombination aus Natriumhydrid und Dimethylsulfoxid das hochreaktive Dimethylsulfinyl-Anion (im Folgenden auch Methylsulfinylmethid, Dimsyl-Anion oder Dimsyl genannt), das eine sehr starke Base darstellt (E. J. Corey, M. Chaykovsky, J. Am. Chem. Soc. 1965, 87, 1345-1353). Durch die hohe Reaktivität dieser Base läuft die Umsetzung von mittel- und langkettigen oder/und mehrwertigen Alkoholen mit aktivierten oder/und nichtaktivierten Halogenbenzonitrilen beim idealen stöchiometrischen Verhältnis ab. Ein Überschuss einer der beiden Komponenten ist in der Regel nicht notwendig. Das ungiftige Dimethylsulfoxid kann im Überschuss eingesetzt werden und somit gleichzeitig als Zweitlösungsmittel dienen. Es kann aber auch problemlos in stöchiometrischen Mengen eingesetzt werden, wenn als Lösungsmittel andere polare aprotische Stoffe wie N,N-Dimethylformamid, N,N-Dimethylacetamid, N-Methylpyrrolidon, N-Ethylpyrrolidon, Sulfolan etc. zugegen sind. Letztere Kombination von Dimsyl in einem polaren aprotischen Lösungsmittel erwies sich aus zwei Gründen als besonders vorteilhaft. Einerseits können Nebenreaktionen, die aufgrund der Zersetzungsneigung von DMSO stattfinden, zurückgedrängt werden und andererseits eine hohe Raum-Zeit-Ausbeute realisiert werden. Wird nämlich Dimsyl direkt in DMSO in situ generiert und die Reaktion in DMSO als alleinigem Lösungsmittel durchgeführt, erfolgt die Umsetzung zwar mit sehr hoher Raum-Zeit-Ausbeute, das resultierende Produkt wird jedoch mit geringer Reinheit erhalten, da zahlreiche Nebenreaktionen ablaufen, die vor allem zu schwefelhaltigen Nebenprodukten führen. Eine aufwändige Aufreinigung und Abtrennung der schwefelhaltigen Nebenprodukte muss erfolgen, wodurch mittels dieser Methode eine großtechnische Anwendung nicht sinnvoll ist. Wird die Reaktion in Abwesenheit von Dimsyl in einem von DMSO verschiedenen polaren aprotischen Lösungsmittel durchgeführt, wird zwar ein Produkt mit sehr hoher Reinheit erhalten; allerdings ist die Raum-Zeit-Ausbeute aufgrund der fehlenden katalytischen Eigenschaften des Dimsyl deutlich ungünstiger. Die Vorteile eines Produkts hoher Reinheit, das mit hoher Raum-Zeit-Ausbeute erhalten wird, kann folglich durch Einsatz geeigneter Mengen Dimsyl in Kombination mit einem von DMSO verschiedenen, polaren aprotischen Lösungsmittel in einfacher Weise realisiert werden.

Die hohe Reaktivität und die stark exotherme Selbstzersetzungsneigung des Dimsyl in DMSO als alleinigem Lösungsmittel bei Temperaturen größer 50 °C stellt zudem auch ein grundsätzliches sicherheitstechnisches Problem dar. So kam es in der Vergangenheit insbesondere bei Scale-Up-Versuchen mehrfach zu schweren Unfällen (z. B. F. A. French, Chem. & Eng. News 1966, 48; G. L. Olson, Chem. & Eng. News 1966, 7.), sodass eine Anwendung bestenfalls im kleinen Maßstab und/oder ohne Erwärmen auf derartige Kopplungen übertragen wurde.

Überraschenderweise wurde nun gefunden, dass insbesondere in Gegenwart von polaren, aprotischen Lösungsmitteln diese Selbstzersetzungsneigung des Dimsyl-Systems (Dimsyl in DMSO) zurückgedrängt werden kann, eine Akkumulation nicht auftritt und somit bei erhöhten Reaktionstemperaturen eine Kopplung von mittel- und langkettigen oder/und mehrwertigen Alkoholen auch mit weniger reaktiven, aber wirtschaftlich bedeutsamen Chlorbenzonitrilen in einer wirtschaftlich günstigen Raum-Zeit-Ausbeute erfolgen kann.

Die Methode ist auf eine Vielzahl an Kombinationen von Halogenbenzonitrilen und Alkoholen anwendbar. Durch Permutation eröffnet sich eine ganze Bibliothek an Verbindungen. Zusätzlich kann beispielsweise eine stufenweise Substitution der Halogensubstituenten erfolgen, indem bei einem gemischten Dihalogenbenzonitril wie 2-Chlor-6-fluorbenzonitril zunächst bei niedriger Temperatur der Fluorsubstituent, bei höherer Temperatur dann der Chlorsubstituent schrittweise mit dem gewünschten Alkohol umgesetzt wird. Auch kann bei mehrwertigen Alkoholen nur ein Teil der Hydroxygruppen gekoppelt werden, wenn das Halogenbenzonitril zusammen mit Dimsyl im Unterschuss eingesetzt wird. Auf diese Weise werden auch komplexere Molekülarchitekturen zugänglich. Auch ringförmige Anordnungen, verzweigte bzw. sternförmige Strukturen etc., Oligomere und Polymere sind denkbar. Einschränkungen ergeben sich lediglich für Substrate, die basenlabil sind oder sich leicht hydrieren lassen. Darüber hinaus können sterisch anspruchsvolle Reste die Umsetzung erschweren oder verhindern.

Im Zusammenhang mit der vorliegenden Erfindung soll dabei unter C1- bis C6-Alkyl ein linearer oder seinerseits alkylsubstituierter Alkylrest mit einer Kettenlänge bis zu 6 Kohlenstoffatomen, insbesondere von 1 bis zu 6 Kohlenstoffatomen, verstanden sein, der insbesondere die allgemeine Formel CₙH₂ₙ₊₁ aufweist, wobei n = eine ganze Zahl von 1 bis 6 bedeutet. Unter Kettenlänge ist dabei die Länge der längsten Kette zu verstehen. Dabei ist insbesondere vorgesehen, dass G1- bis C6-Alkyl Methyl, Ethyl, Propyl, Butyl, Pentyl oder Hexyl bedeutet, wobei diese Alkylreste weiterhin bevorzugt unverzweigt oder ihrerseits einfach oder mehrfach alkylsubstituiert sein können. Sollte ein C1- bis C6-Alkylrest seinerseits alkylsubstituiert sein, so sind C1- bis C6-Alkylreste bevorzugt, die ihrerseits mit C1- bis C5-Alkyl substituiert sind. Dabei kann C1- bis C5-Alkyl weiterhin bevorzugt Methyl, Ethyl, n-Propyl, n-Butyl oder n-Pentyl bedeuten.

Im Zusammenhang mit der vorliegenden Erfindung soll weiterhin unter C1- bis C6-Hydoxyalkyl ein linearer oder seinerseits alkylsubstituierter Hydoxyalkylrest mit einer Kettenlänge bis zu 6 Kohlenstoffatomen, insbesondere von 1 bis zu 6 Kohlenstoffatomen, verstanden sein, der mit mindestens einer Hydroxygruppe substituiert ist und der insbesondere die allgemeine Formel CₙH₍₂ₙ₊₁₎₋ₚ(OH)ₚ aufweist, wobei p = eine ganze Zahl von 1 bis 6 bedeutet und p ≤ n gilt. Unter Kettenlänge ist dabei die Länge der längsten Kette zu verstehen. Dabei ist insbesondere vorgesehen, dass C1- bis C6-Hydroxyalkyl Hydroxymethyl, 2-Hydroxyethyl, 3-Hydroxypropyl, 4-Hydroxybutyl, 5-Hydroxypentyl oder 6-Hydroxyhexyl bedeutet, wobei diese Alkylreste weiterhin bevorzugt unverzweigt oder ihrerseits einfach oder mehrfach alkylsubstituiert sein können. Sollte ein C1- bis C6-Hydroxyalkylrest seinerseits alkylsubstituiert sein, so sind C1- bis C6-Hydroxyalkylreste bevorzugt, die ihrerseits mit C1- bis C5-Alkyl substituiert sind. Dabei kann C1- bis C5-Alkyl weiterhin bevorzugt Methyl, Ethyl, n-Propyl, n-Butyl oder n-Pentyl bedeuten.

Gemäß der vorliegenden Erfindung kann (R¹ und R²) oder (R¹ und R⁴) gemäß Formel (I) oder Formel (IV) auch gemeinsam C1- bis C6-Alkylen oder C1- bis C6-Hydroxyalkylen bedeuten, wobei (R¹ und R²) oder (R¹ und R⁴) mit den weiteren Kohlenstoffatomen des Alkohols oder der Alkoxy-Seitenkette des Benzonitrils einen Ring bilden. Dabei soll unter C1- bis C6-Alkylen ein zweiwertiger Alkylrest mit einer Kettenlänge bis zu 6 Kohlenstoffatomen, insbesondere von 1 bis zu 6 Kohlenstoffatomen, verstanden sein, der insbesondere die allgemeine Formel CₙH₂ₙ aufweist, wobei n = eine ganze Zahl von 1 bis 6 bedeutet. Dabei soll unter C1- bis C6-Hydoxyalkylen ein zweiwertiger Hydroxyalkylrest mit einer Kettenlänge von 1 bis zu 6 Kohlenstoffatomen verstanden sein, der mindestens eine und höchstens 6 Hydroxygruppen aufweist und der insbesondere die allgemeine Formel CₙH₂ₙ₋ₚ(OH)ₚ mit n = eine ganze Zahl von 1 bis 6 und p ≤ n aufweist. Insbesondere kann hierbei vorgesehen sein, dass (R¹ und R²) oder (R¹ und R⁴) gemeinsam Methylen, Ethylen, Propylen, Butylen, Pentylen, Hexylen, Hydroxymethylen, Hydroxyethylen, Hydroxypropylen, Hydroxybutylen, Hydroxypentylen oder Hydroxyhexylen bedeuten, wobei diese Alkylenreste oder Hydroxyalkylenreste ihrerseits gegebenenfalls einfach oder mehrfach alkylsubstituiert sein können. Sollte ein C1- bis C6-Alkylen oder ein C1- bis C6-Hydroxyalkylen seinerseits alkylsubstituiert sein, so sind C1- bis C6-Alkylen und C1- bis C6-Hydroxyalkylen bevorzugt, die ihrerseits mit C1- bis C5-Alkyl gemäß der oben genannten Bedeutung substituiert sind. Dabei bildet (R¹ und R²) oder (R¹ und R⁴) gemeinsam mit den weiteren Kohlenstoffatomen des Alkohols oder der Alkoxy-Seitenkette des Benzonitrils einen gesättigten, cycloaliphatischen Ring, der seinerseits gegebenenfalls einfach oder mehrfach mit C1- bis C5-Alkylresten der oben wiedergegebenen Bedeutung substituiert sein kann.

Des Weiteren soll unter Natrium-methylsulfinylmethid eine ionische Verbindung verstanden sein, die aus einem Natriumhydrid und Dimethylsulfoxid gebildet wird, und die gemäß IUPAC-Nomenklatur als Sodium Methylsulfinylmethylide bezeichnet wird.

Dabei ist im Zusammenhang mit der vorliegenden Erfindung unter einem polaren, aprotischen Lösungsmittel jedwedes organische Lösungsmittel zu verstehen, das definitionsgemäß verschieden ist von DMSO, eine Permittivität von über 15 aufweist und in der Lage ist, Kationen zu solvatisieren.

Erfindungswesentlich wird die Reaktion in Gegenwart von Natrium-methylsulfinylmethid in einem polaren aprotischen Lösungsmittel durchgeführt, das verschieden ist von Dimethylsulfoxid. Besonders bevorzugt wird hierbei das Natrium-methylsulfinylmethid im stöchiometrischen Verhältnis zu den in der Reaktion eingesetzten Alkoholen oder in äquivalenten Mengen zu der zur Reaktion zu bringenden Anzahl an Alkoholgruppen, insbesondere Hydroxygruppen, eingesetzt. Solchenfalls wird kein Dimethylsulfoxid als Lösungsmittel eingesetzt. Es kann jedoch auch Dimethylsulfoxid als Zweitlösungsmittel neben dem polaren, aprotischen Lösungsmittel eingesetzt werden. Hierbei ist gemäß einer besonders bevorzugten Ausführung vorgesehen, dass als polares aprotisches Lösungsmittel ein Lösungsmittelgemisch aus mindestens einem polaren, aprotischen Lösungsmittel und DMSO, das weiterhin bevorzugt a) 60 bis 100 Gew.-% mindestens eines polaren aprotischen Lösungsmittels, das verschieden ist von Dimethylsulfoxid, und b) 0 bis 40 Gew.-% Dimethylsulfoxid enthält, zum Einsatz gebracht wird. Besonders bevorzugt ist dabei ein Lösungsmittelgemisch, das aus a) 75 bis 100 Gew.-%, weiterhin bevorzugt 85 bis 100 Gew.-%, mindestens eines polaren aprotischen Lösungsmittels, das verschieden ist von Dimethylsulfoxid, und b) 0 bis 25 Gew.-%, weiterhin bevorzugt 0 bis 15 Gew.-%, Dimethylsulfoxid besteht.

Gleichzeitig oder unabhängig hiervon kann weiterhin bevorzugt als polares, aprotisches Lösungsmittel ein Lösungsmittel oder ein Lösungsmittelgemisch verwendet werden, das mindestens ein Lösungsmittel ausgewählt aus der Gruppe N,N-Dimethylformamid, N,N-Dimethylacetamid, N-Methylpyrrolidon, N-Ethylpyrrolidon oder Sulfolan umfasst. Besonders bevorzugt umfasst das polare, aprotische Lösungsmittel N,N-Dimethylformamid. Der Einsatz dieser Lösungsmittel gewährleistet eine besonders hohe Raum-Zeit-Ausbeute und ermöglicht die Isolierung der gewünschten Produkte ohne aufwändige Aufarbeitung der Rohprodukte als Endprodukte.

Gemäß einer Weiterbildung der Erfindung kann dabei auch vorgesehen sein, dass das Natrium-methylsulfinylmethid in dem polaren, aprotischen Lösungsmittel in situ hergestellt wird. In diesem Fall kann DMSO als Reagenz in dem polaren, aprotischen Lösungsmittel vorgelegt und Natriumhydrid, insbesondere portionsweise und weiterhin bevorzugt unter Schutzgasatmosphäre, zugegeben werden. Es kann jedoch auch vorgesehen sein, dass in einem ersten Verfahrensschritt das DMSO als Reagenz und das polare, aprotische Lösungsmittel in einem Reaktionsgefäß vorgelegt werden, in einem zweiten Reaktionsschritt der Alkohol oder/und das Halogenbenzonitril in dem vorgelegten Gemisch gelöst oder/und suspendiert werden und in einem dritten Verfahrensschritt Natriumhydrid dem Reaktionsgemisch zur Bildung des Natrium-methylsulfinylmethid zugefügt wird. Somit ist auch ein Verfahren Gegenstand der vorliegenden Erfindung, in dem das Natrium-methylsulfinylmethid in situ in dem Reaktionsgemisch oder in situ durch Zugabe von Natriumhydrid zum Reaktionsgemisch hergestellt wird.

Ganz besonders bevorzugt kann in dem Reaktionsgemisch das Natrium-methylsulfinylmethid in einem molaren Verhältnis Natrium-methylsulfinylmethid zu den in der Reaktion eingesetzten Alkoholen oder zu den in der Reaktion eingesetzten Hydroxygruppen oder zu der zur Reaktion zu bringenden Anzahl an Hydroxygruppen im Bereich von 1,5 : 1 bis 1 : 1, insbesondere im Bereich von 1,3 : 1 bis 1: 1 und ganz besonders bevorzugt von 1,2 : 1 bis 1 : 1 und am meisten bevorzugt von 1 : 1 eingesetzt werden, wobei weiterhin bevorzugt neben dem zur Bildung von Natrium-methylsulfinylmethid notwendigen DMSO kein DMSO als Lösungsmittel eingesetzt wird.

In einer weiteren Ausführung des erfindungsgemäßen Verfahrens kann die Reaktion bei einer Temperatur von 0 bis 150 °C, insbesondere von 20 bis 150 °C und ganz besonders bevorzugt bei 50 bis 150 °C erfolgen. Hierbei hat sich besonders überraschend gezeigt, dass bei Temperaturen oberhalb von 50 °C kaum Nebenreaktionen stattfinden. Somit kann ein Verfahren bereitgestellt werden, mit welchem Produkte erhalten werden, die ohne großtechnische Aufarbeitung eine relativ hohe Reinheit aufweisen.

Wie bereits ausgeführt, kann die vorliegende Erfindung auf eine Vielzahl an Reaktionen übertragen werden. Gemäß einer bevorzugten Ausführung der vorliegenden Erfindung können jedoch insbesondere Halogenbenzonitrile gemäß Formel (III) eingesetzt werden, für die gleichzeitig oder unabhängig voneinander gilt:
X = Fluor oder Chlor;
Y = Wasserstoff oder Chlor.

Ganz besonders bevorzugt können Chlorbenzonitrile (Formel (III): X=Chlor, Y = Wasserstoff) oder Dichlorbenzonitrile (Formel (III): X, Y = Chlor) eingesetzt werden. Dabei können weiterhin bevorzugt monochlorierte Benzonitrile (Formel (III): X = Chlor, Y = Wasserstoff) eingesetzt werden, die in 2 oder 4-Stellung zur Nitrilgruppe chloriert sind, somit o-Chlorbenzonitril oder p-Chlorbenzonitril. Die Herstellung dieser Verbindungen ist aus der Literatur gut bekannt.

Besonders bevorzugt können in dem erfindungsgemäßen Verfahren mittel- oder langkettige Alkohole oder/und mehrwertige Alkohole zum Einsatz gebracht werden. Insbesondere können dabei Alkohole mit einer Kohlenstoffkette mit 4 oder mehr Kohlenstoffatomen zum Einsatz gebracht werden.

Gemäß einer weiter bevorzugten Ausführung der vorliegenden Erfindung können insbesondere auch Alkohole gemäß Formel (IV) eingesetzt werden, für die gleichzeitig oder unabhängig voneinander gilt:
R¹, R², R³, R⁴ = gleichzeitig oder unabhängig voneinander Wasserstoff oder C1-C6-Alkyl;
m, n, o = gleichzeitig oder unabhängig voneinander eine ganze Zahl von 1 bis 4;
R⁵ = Wasserstoff, Hydroxy oder
   ein Rest gemäß Formel (II) mit Y = Wasserstoff oder Chlor und p = 0.

Ganz besonders bevorzugt können in dem erfindungsgemäßen Verfahren Alkohole ausgewählt aus der Gruppe 1-Hexanol, 2,2-Dimethylpropan-1,3-diol, Cyclohexanol, 1,2-Ethandiol, 1,3-Propandiol, 1,4-Butandiol, 1,5-Pentandiol, 1,6-Hexandiol, 1,12-Dodecandiol, 12-(4-Cyanophenoxy)dodecan-1-ol oder Pentaerythrit eingesetzt werden.

### Beispiele:

### Allgemeine Herstellvorschrift für erfindungsgemäße Alkoxybenzonitrile:

Unter einer Schutzgasatmosphäre werden bei Raumtemperatur Alkohol und Nitril in einem polaren, aprotischen Lösungsmittel gelöst und/oder suspendiert. Zu dieser Mischung wird unter Rühren die angegebene Menge DMSO hinzugefügt und auf 50-150 °C erhitzt. Natriumhydrid wird in kleinen Portionen als 60 %ige Suspension in Mineralöl zugegeben, so dass sich Natrium-methylsulfinylmethid bilden kann. Nach beendeter Zugabe wird das Reaktionsgemisch 1h bis 18 h bei 50-150 °C gerührt. Es wird auf Raumtemperatur abgekühlt und das Produkt unter Rühren durch Zugabe von Wasser ausgefällt. Das Rohprodukt wird abfiltriert, gewaschen und im Vakuum getrocknet.

### Beispiel 1:

### 1,2-Bis(4-cyanophenoxy)ethan

| | | | | |
|---|---|---|---|---|
| Ansatzgröße: | 1,2-Ethandiol | | 1,55 g | 25 mmol |
| | p-Chlorbenzonitril | | 6,88 g | 50 mmol |
| | Natriumhydrid | | 2,00 g | 50 mmol |
| | Dimethylsulfoxid | | 3,91 g | 50 mmol |
| | N,N-Dimethylformamid | | 40 ml | |
| | | | | |
| Ausbeute: | 3,2 g (48 %) beiger Feststoff | | | |
| Schmelztemperatur: | 201,0 °C | | | |
| | | | | |
| ¹H-NMR (CDCl₃, RT): | *δ*[ppm] = 7,59 (d, ³*J* = 7,0 Hz, 4H, Ar-H), 6,98 (d, ³*J* = 6,5 Hz, 4H, Ar-H), 4,38 (s, 4H, CH₂). | | | |
| ¹³C-NMR (CDCl₃, RT): | *δ* [ppm] = 161,6, 134,1, 119,0, 115,3, 104,8, 66,5. | | | |
| Elementaranalyse: | berechnet | C, 72,72 % | H, 4,58 %, | N, 10,60 % S, - |
| | gefunden | C, 72,10 % | H, 4,51 %, | N, 10,66 % S, 0,03 % |

### Beispiel 2:

### 1,3-Bis(4-cyanophenoxy)propan

| | | | | |
|---|---|---|---|---|
| Ansatzgröße: | 1,3-Propandiol | | 1,90 g | 25 mmol |
| | p-Chlorbenzonitril | | 6,88 g | 50 mmol |
| | Natriumhydrid | | 2,00 g | 50 mmol |
| | Dimethylsulfoxid | | 3,91 g | 50 mmol |
| | N,N-Dimethylformamid | | 40 ml | |
| Ausbeute: | 2,6 g (37 %) beiger Feststoff | | | |
| Schmelztemperatur: | 187,4 °C | | | |
| | | | | |
| ¹H-NMR (CDCl₃, RT): | *δ*[ppm] = 7,56 (d, ³*J* = 8,0 Hz, 4H, Ar-H), 6,93 (d, ³*J* = 8,0 Hz, 4H, Ar-H), 4,19 (t, ³*J* = 6,0 Hz, 4H, CH₂), 2,30 (quin, ³*J* = 6,0 Hz, 2H, CH₂). | | | |
| ¹³C-NMR (CDCl₃, RT): | *δ*[ppm] = 162,0, 134,0, 119,1, 115,2, 104,3, 64,4, 28,8. | | | |
| Elementaranalyse: | berechnet | C, 73,37 % | H, 5,07 %, | N, 10,07 % |
| | gefunden | C, 71,98 % | H, 5,04 %, | N, 10,12 % |

### Beispiel 3:

### 1,4-Bis(4-cyanophenoxy)butan

| | | | | |
|---|---|---|---|---|
| Ansatzgröße: | 1,4-Butandiol | | 2,25 g | 25 mmol |
| | p-Chlorbenzonitril | | 6,88 g | 50 mmol |
| | Natriumhydrid | | 2,00 g | 50 mmol |
| | Dimethylsulfoxid | | 3,91 g | 50 mmol |
| | N,N-Dimethylformamid | | 40 ml | |
| | | | | |
| Ausbeute: | 3,0 g (41 %) beiger Feststoff | | | |
| Schmelztemperatur: | 166,2 °C | | | |
| | | | | |
| ¹H-NMR (CDCl₃, RT): | *δ*[ppm] = 7,56 (d, ³*J* = 8,5 Hz, 4H, Ar-H), 6,92 (d, ³*J* = 9,0 Hz, 4H, Ar-H), 4,07 (m, 4H, CH₂), 1,99 (m, 4H, CH₂). | | | |
| ¹³C-NMR (CDCl₃, RT): | *δ*[ppm] = 162,2, 134,0, 119,2, 115,1, 104,0, 67,7, 25,7. | | | |
| Elementaranalyse: | berechnet | C, 73,95 % | H, 5,52 %, | N, 9,58 % S,- |
| | gefunden | C, 73,35 % | H, 5,45 %, | N, 9,69 % S, 0,01 % |

### Beispiel 4:

### 1,5-Bis(4-cyanophenoxy)pentan

| | | | | |
|---|---|---|---|---|
| Ansatzgröße: | 1,5-Pentandiol | | 2,60 g | 25 mmol |
| | p-Chlorbenzonitril | | 6,88 g | 50 mmol |
| | Natriumhydrid | | 2,00 g | 50 mmol |
| | Dimethylsulfoxid | | 3,91 g | 50 mmol |
| | N,N-Dimethylformamid | | 40 ml | |
| | | | | |
| Ausbeute: | 3,5 g (46 %) beiger Feststoff | | | |
| Schmelztemperatur: | 107,8 °C | | | |
| | | | | |
| ¹H-NMR (CDCl₃, RT): | *δ*[ppm] = 7,55 (d, ³*J* = 6,5 Hz, 4H, Ar-H), 6,92 (d, ³*J* = 10 Hz, 4H, Ar-H), 4,02 (t, ³*J* = 6,5 Hz, 4H, CH₂), 1,86 (m, 4H, CH₂), 1,65 (m, 2H, CH₂). | | | |
| ¹³C-NMR (CDCl₃, RT): | *δ*[ppm] = 162,3, 134,0, 119,2, 115,1, 103,9, 68,0, 28,7, 22,7. | | | |
| Elementaranalyse: | berechnet | C, 74,49 % | H, 5,92 %, | N, 9,14 % |
| | gefunden | C, 73,80 % | H, 5,76 %, | N, 8,97 % |

### Beispiel 5:

### 1,6-Bis(2-cyanophenoxy)hexan

| | | | | | |
|---|---|---|---|---|---|
| Ansatzgröße: | 1,6-Hexandiol | | 2,95 g | 25 mmol | |
| | o-Chlorbenzonitril | | 6,88 g | 50 mmol | |
| | Natriumhydrid | | 2,00 g | 50 mmol | |
| | Dimethylsulfoxid | | 3,91 g | 50 mmol | |
| | N,N-Dimethylformamid | | 40 ml | | |
| | | | | | |
| Ausbeute: | 3,1 g (39 %) beiger Feststoff | | | | |
| Schmelztemperatur: | 97,8 °C | | | | |
| | | | | | |
| ¹H-NMR (CDCl₃, RT): | *δ*[ppm] = 7,50 (m, 4H, Ar-H), 6,96 (m, 4H, Ar-H), 4,08 (t, ³*J* = 6,3 Hz, 4H, CH₂), 1,88 (m, 4H, CH₂), 1,60 (m, 4H, CH₂). | | | | |
| ¹³C-NMR (CDCl₃, RT): | *δ*[ppm] = 160,7, 134,3, 133,6, 120,5, 116,5, 112,2, 101,9, 68,7, 28,6, 25,4. | | | | |
| Elementaranalyse: | berechnet | C, 74,98 % | H, 6,29 %, | N, 8,74 % | S, - |
| | gefunden | C, 74,50 % | H, 6,42 %, | N, 8,77 % | S, 0,09 % |

### Beispiel 6:

### 1,12-Bis(4-cyanophenoxy)dodecan

| | | | | |
|---|---|---|---|---|
| Ansatzgröße: | 1,12-Dodecandiol | | 5,06 g | 25 mmol |
| | p-Chlorbenzonitril | | 6,88 g | 50 mmol |
| | Natriumhydrid | | 2,00 g | 50 mmol |
| | Dimethylsulfoxid | | 3,91 g | 50 mmol |
| | N,N-Dimethylformamid | | 40 ml | |
| Ausbeute: | 7,7 g (76 %) beiger Feststoff | | | |
| Schmelztemperatur: | 111,1 °C | | | |
| | | | | |
| ¹H-NMR (CDCl₃, RT): | *δ*[ppm] = 7,54 (d, ³*J* = 8,5 Hz, 4H, Ar-H), 6,90 (d, ³*J* = 9 Hz, 4H, Ar-H), 3,97 (t, ³*J* = 6,5 Hz, 4H, CH₂), 1,77 (m, 4H, CH₂), 1,43 (m, 4H, CH₂), 1,2-1,1 (m, 12H, CH₂). | | | |
| ¹³C-NMR (CDCl₃, RT): | *δ*[ppm] = 162,4, 133,9, 119,2, 115,1, 103,6, 68,3, 29,5, 29,4, 29,2, 28,9, 25,9. | | | |
| Elementaranalyse: | berechnet | C, 77,19 % | H, 7,97 %, | N, 6,92 % |
| | gefunden | C, 77,09 % | H, 8,04 %, | N, 6,94 % |

### Beispiel 7:

### 1-(2-cyanophenoxy)-12-(4-cyanophenoxy)dodecan

| | | | | |
|---|---|---|---|---|
| Ansatzgröße: | 12-(4-cyanophenoxy)dodecan-1-ol | | 7,59 g | 25 mmol |
| | o-Chlorbenzonitril | | 6,88 g | 50 mmol |
| | Natriumhydrid | | 1,00 g | 25 mmol |
| | Dimethylsulfoxid | | 1,95 g | 25 mmol |
| | N,N-Dimethylformamid | | 40 ml | |
| Ausbeute: | 4,4 g (44 %) | | | |
| Schmelztemperatur: | 77,5 °C | | | |
| ¹H-NMR (CDCl₃, RT): | *δ*[ppm] = 7,5 (m, 4H, Ar-H), 6,9 (m, 4H, Ar-H), 4,04 (t, ³*J* = 6,8 Hz, 2H, CH₂), 3,97 (t, ³*J* = 6,5 Hz, 2H, CH₂), 1,9 (m, 4H, CH₂), 1,5 (m, 4H, CH₂), 1,4-1,2 (m, 12H, CH₂). | | | |
| ¹³C-NMR (CDCl₃, RT): | *δ*[ppm] = 162,4, 160,8, 134,2, 134,0, 133,9, 133,8, 133,7, 130,0, 127,1, 120,5, 119,3, 116,5, 115,2, 112,2, 103,6, 102,0, 69,01, 68,39, 29,65, 29,47, 29,44, 29,37, 29,32, 29,26, 29,24, 28,92, 28,87, 25,86, 25,85, 25,80. | | | |
| Elementaranalyse: | berechnet | C, 77,19 % | H, 7,97 %, | N, 6,92 % |
| | gefunden | C, 77,34 % | H, 8,02 %, | N, 6,69 % |

### Beispiel 8:

### 1,2,3,4-Tetrakis(4-cyanophenoxy)neopentan

| | | | | |
|---|---|---|---|---|
| Ansatzgröße: | Pentaerythrit | | 3,40 g | 25 mmol |
| | p-Chlorbenzonitril | | 13,8 g | 100 mmol |
| | Natriumhydrid | | 4,00 g | 100 mmol |
| | Dimethylsulfoxid | | 7,81 g | 100 mmol |
| | N,N-Dimethylformamid | | 40 ml | |
| | | | | |
| Ausbeute: | 5,0 g (37 %) | | | |
| Schmelztemperatur: | ca. 200 °C (Zersetzung) | | | |
| | | | | |
| ¹H-NMR (CDCl₃, RT): | *δ*[ppm] = 7,56 (d, ³*J* = 6,5 Hz, 4H, Ar-H), 6,95 (d, ³*J* = 7,0 Hz, 4H, Ar-H), 4,38 (s, 8H, CH₂). | | | |
| ¹³C-NMR (CDCl₃, RT): | *δ*[ppm] = 161,3, 134,0, 119,7, 115,2, 105,0, 66,3, 44,5. | | | |
| Elementaranalyse: | berechnet | C, 73,32 % | H, 4,48 %, | N, 10,36 % |
| | gefunden | C, 71,97 % | H, 4,49 %, | N, 10,41 % |

### Angepasste Herstellvorschrift 1 für Beispiel 9 bis 12:

Abweichend von der allgemeinen Herstellvorschrift wird die Quenche nach der Zugabe von Wasser mit Diethylether oder Toluol versetzt und die organische Phase abgetrennt. Die wässrige Phase wird mehrmals mit dem organischen Lösungsmittel extrahiert. Die vereinten organischen Phasen werden mit Natriumsulfat getrocknet. Leicht flüchtige Anteil werden am Rotationsverdampfer entfernt. Das Produkt wird nach Säulenchromatographie an Kieselgel erhalten.

### Beispiel 9:

### (4-Cyanophenoxy)cyclohexan

| | | | | |
|---|---|---|---|---|
| Ansatzgröße: | Cyclohexanol | | 5,08 g | 50 mmol |
| | p-Chlorbenzonitril | | 6,88 g | 50 mmol |
| | Natriumhydrid | | 2,00 g | 50 mmol |
| | Dimethylsulfoxid | | 3,91 g | 50 mmol |
| | N,N-Dimethylformamid | | 40 ml | |
| Ausbeute: | 3,1 g (31 %) gelbliches Öl | | | |
| ¹H-NMR (CDCl₃, RT): | *δ*[ppm] = 7,49 (d, ³*J* = 9,3 Hz, 2H, Ar-H), 6,87 (d, ³*J* = 9,5 Hz, 2H, Ar-H), 4,28 (m, 1H, CH), 1,90 (m, 2H, CH₂), 1,74 (m, 2H, CH₂), 1,49 (m, 3H, CH₂), 1,34 (m, 3H, CH₂). | | | |
| ¹³C-NMR (CDCl₃, RT): | *δ*[ppm] = 161,1, 133,8, 119,2, 116,0, 103,1, 75,5, 31,3, 25,3, 23,4. | | | |
| Elementaranalyse: | berechnet | C, 77,58 % | H, 7,51 %, | N, 6,96 % |
| | gefunden | C, 77,70 % | H, 7,56 %, | N, 6,92 % |

### Beispiel 10:

### 1-(4-Cyanophenoxy)hexan

| | | | | |
|---|---|---|---|---|
| Ansatzgröße: | 1-Hexanol | | 5,11 g | 50 mmol |
| | p-Chlorbenzonitril | | 6,88 g | 50 mmol |
| | Natriumhydrid | | 2,00 g | 50 mmol |
| | Dimethylsulfoxid | | 3,91 g | 50 mmol |
| | N,N-Dimethylformamid | | 40 ml | |
| Ausbeute: | 3,8 g (37 %) farbloses Öl | | | |
| ¹H-NMR (CDCl₃, RT): | *δ*[ppm] = 7,53 (d, ³*J* = 8,8 Hz, 2H, Ar-H), 6,90 (d, ³*J* = 7,0 Hz, 2H, Ar-H), 3,97 (t, ³*J* = 6,5 Hz, 2H, CH₂), 1,77 (m, 2H, CH₂), 1,43 (m, 2H, CH₂), 1,32 (m, 4H, CH₂), 0,88 (m, 3H, CH₃). | | | |
| ¹³C-NMR (CDCl₃, RT): | *δ*[ppm] = 162,4, 133,9, 119,3, 115,2, 103,6, 68,4, 31,5, 28,9, 25,7, 22,5, 13,9. | | | |
| Elementaranalyse: | berechnet | C, 76,81 % | H, 8,43 %, | N, 6,89 % |
| | gefunden | C, 77,43 % | H, 9,01 %, | N, 6,61 % |

### Beispiel 11:

### 1,3-Bis-(4-cyanophenoxy)-2,2-dimethyl propan

| | | | | |
|---|---|---|---|---|
| Ansatzgröße: | 2,2-Dimethylpropan-1,3-diol | | 2,60 g | 25 mmol |
| | p-Chlorbenzonitril | | 6,88 g | 50 mmol |
| | Natriumhydrid | | 2,00 g | 50 mmol |
| | Dimethylsulfoxid | | 3,91 g | 50 mmol |
| | N,N-Dimethylformamid | | 40 ml | |
| Ausbeute: | 4,2 g (55 %) farbloser Feststoff | | | |
| Schmelztemperatur: | 109,5 °C | | | |
| ¹H-NMR (CDCl₃, RT): | *δ*[ppm] = 7,52 (d, ³*J* = 9,0 Hz, 4H, Ar-H), 6,92 (t, ³*J* = 9,0 Hz, 4H, Ar-H), 3,86 (s, 4H, CH₂), 1,15 (s, 6H, CH₃). | | | |
| ¹³C-NMR (CDCl₃, RT): | *δ*[ppm] = 162,2, 133,9, 119,1, 115,2, 104,0, 73,1, 35,7, 21,8. | | | |
| Elementaranalyse: | berechnet | C, 74,49 % | H, 5,92 %, | N, 9,14 % |
| | gefunden | C, 74,50 % | H, 6,17 %, | N, 9,02 % |

### Beispiel 12:

### 12-(4-cyanophenoxy)dodecan-1-ol

| | | | | |
|---|---|---|---|---|
| Ansatzgröße: | 1,12-Dodecandiol | | 10,1 g | 50 mmol |
| | p-Chlorbenzonitril | | 6,88 g | 50 mmol |
| | Natriumhydrid | | 2,00 g | 50 mmol |
| | Dimethylsulfoxid | | 3,91 g | 50 mmol |
| | N,N-Dimethylformamid | | 40 ml | |
| Ausbeute: | 7,7 g (51 %) weißes Pulver | | | |
| Schmelztemperatur: | 88,7 °C | | | |
| ¹H-NMR (CDCl₃, RT): | *δ*[ppm] = 7,54 (d, *³J* = 7,0 Hz, 2H, Ar-H), 6,91 (d, ³*J* = 7,0 Hz, 2H, Ar-H), 3,97 (t, ³*J* = 6,5 Hz, 2H, CH₂), 3,61 (t, ³*J* = 6,5 Hz, 2H, CH₂), 1,77 (m, 2H, CH₂), 1,61 (s, 1H, OH), 1,54 (m, 2H, CH₂), 1,41 (m, 2H, CH₂), 1,2-1,1 (m, 14H, CH₂). | | | |
| ¹³C-NMR (CDCl₃, RT): | *δ*[ppm] = 162,5, 133,9, 119,3, 115,2, 103,6, 68,4, 63,1, 32,8, 29,58, 29,53, 29,50, 29,4, 29,3, 29,0, 25,9, 25,4. | | | |
| Elementaranalyse: | berechnet | C, 75,21 % | H, 9,63 %, | N, 4,62 % |
| | gefunden | C, 74,78 % | H, 9,44 %, | N, 4,51 % |

### Angepasste Herstellvorschrift 2 für das Beispiel 13:

Abweichend von der allgemeinen Herstellvorschrift und abweichend von der angepassten Herstellvorschrift 1 wird Natriumhydrid bei 0 °C dosiert und die Reaktion bei Raumtemperatur durchgeführt.

### Beispiel 13:

### 6-(3-Chlor-2-cyanophenoxy)-hexan-1-ol

| | | | |
|---|---|---|---|
| Ansatzgröße: | 1,6-Hexandiol | 47,27 g | 400 mmol |
| | 2-Chlor-6-fluorbenzonitril | 15,56 g | 100 mmol |
| | Natriumhydrid | 4,00 g | 100 mmol |
| | Dimethylsulfoxid | 7,81 g | 100 mmol |
| | N,N-Dimethylformamid | 200 ml | |
| Ausbeute: | 17,3 g (68 %) farbloses Öl | | |
| ¹H-NMR (CDCl₃, RT): | *δ*[ppm] = 7,35 (t, ³*J* = 8,5 Hz, 1H, Ar-H), 6,95 (d, ³*J* = 8,0 Hz, 1H, Ar-H), 6,80 (d, ³*J* = 8,0 Hz, 1H, Ar-H), 4,01 (t, ³*J* = 6,5 Hz, 2H, CH₂), 3,56 (t, ³*J* = 6,5 Hz, 2H, CH₂), 2,36 (s, 1H, OH), 1,78 (m, 2H, CH₂), 1,52 (m, 2H, CH₂), 1,45 (m, 2H, CH₂), 1,37 (m, 2H, CH₂). | | |
| ¹³C-NMR (CDCl₃, RT): | *δ*[ppm] = 161,8, 137,1, 134,1, 121,0, 113,2, 110,1, 102,4, 69,2, 61,8, 32,1, 28,3, 25,2, 25,0. | | |

### Vergleichsbeispiel 1:

### 1,6-Bis(4-cyanophenoxy)hexan

Unter einer Schutzgasatmosphäre werden bei Raumtemperatur 2,95 g (25 mmol) 1,6-Hexandiol und 6,88 g (50 mmol) p-Chlorbenzonitril in 40 ml Dimethylsulfoxid (DMSO) gelöst. Die Mischung wird unter Rühren auf 70 °C erhitzt. Innerhalb von zehn Minuten werden 2,20 g (55 mmol) Natriumhydrid (60 %ige Suspension in Mineralöl) portionsweise zugegeben. Das Reaktionsgemisch wird sechs Stunden bei 70 °C gerührt.Im Wasserbad wird auf 30 °C abgekühlt und unter Rühren durch langsame Zugabe von 100 ml Wasser das Produkt gefällt und die tiefbraune Suspension filtriert. Der braune Filterkuchen wird zweimal mit je 25 ml heißem Wasser, einmal mit 10 ml Isopropanol sowie einmal mit 10 ml Cyclohexan gewaschen. Das braune Produkt wird im Vakuum bei 70 °C getrocknet.

| | | | | | |
|---|---|---|---|---|---|
| Ausbeute: | 7,30 g brauner Feststoff (91,1 % der Theorie) | | | | |
| Gehalt (GC): | 86,1 Flächen-% | | | | |
| Reinausbeute: | 78,4 % 1,6-*Bis*(4-cyanophenoxy)hexan | | | | |
| Elementaranalyse: | berechnet | C, 74,98 % | H, 6,29 %, | N, 8,74 % | S, - |
| | gefunden | C, 73,49 % | H, 6,60 %, | N, 8,13 % | S, 0,90 % |

### Vergleichsbeispiel 2:

### 1,6-Bis(4-cyanophenoxy)hexan

Unter einer Schutzgasatmosphäre werden bei Raumtemperatur 2,95 g (25 mmol) 1,6-Hexandiol und 6,88 g (50 mmol) p-Chlorbenzonitril in 40 ml N-Methylpyrrolidon (NMP) gelöst. Die Mischung wird unter Rühren auf 70 °C erhitzt. Innerhalb von zehn Minuten werden 2,20 g (55 mmol) Natriumhydrid (60 %ige Suspension in Mineralöl) portionsweise zugegeben. Das Reaktionsgemisch wird sechs Stunden bei 70 °C gerührt. Im Wasserbad wird auf 30 °C abgekühlt und unter Rühren durch langsame Zugabe von 100 ml Wasser das Produkt gefällt und die hellbraune Suspension filtriert. Der hellbraune Filterkuchen wird zweimal mit je 25 ml heißem Wasser, einmal mit 10 ml Isopropanol sowie einmal mit 10 ml Cyclohexan gewaschen. Das beige Produkt wird im Vakuum bei 70 °C getrocknet.

| | | | | | |
|---|---|---|---|---|---|
| Ausbeute: | 4,60 g beiger Feststoff (57,4 % der Theorie) | | | | |
| Gehalt (GC): | 99,2 Flächen-% | | | | |
| Reinausbeute: | 56,9 % 1,6-*Bis*(4-cyanophenoxy)hexan | | | | |
| Elementaranalyse: | berechnet | C, 74,98 % | H, 6,29 %, | N, 8,74 % | S, - |
| | gefunden | C, 74,57 % | H, 6,42 %, | N, 8,64 % | S, <0,01 % |

### Beispiel 14:

### 1,6-Bis(4-cyanophenoxy)hexan

Unter einer Schutzgasatmosphäre werden bei Raumtemperatur 2,95 g (25 mmol) 1,6-Hexandiol 6,88 g (50 mmol) p-Chlorbenzonitril und 3,91 g (50 mmol) Dimethylsulfoxid (DMSO) in 40 ml N-Methylpyrrolidon (NMP) gelöst. Die Mischung wird unter Rühren auf 70 °C erhitzt. Innerhalb von zehn Minuten werden 2,20 g (55 mmol) Natriumhydrid (60 %ige Suspension in Mineralöl) portionsweise zugegeben. Das Reaktionsgemisch wird sechs Stunden bei 70 °C gerührt.

Im Wasserbad wird auf 30 °C abgekühlt und unter Rühren durch langsame Zugabe von 100 ml Wasser das Produkt gefällt und die hellbraune Suspension filtriert. Der hellbraune Filterkuchen wird zweimal mit je 25 ml heißem Wasser, einmal mit 10 ml Isopropanol sowie einmal mit 10 ml Cyclohexan gewaschen. Das beige Produkt wird im Vakuum bei 70 °C getrocknet.

| | | | | | |
|---|---|---|---|---|---|
| Ausbeute: | 7,00 g beiger Feststoff (87,4 % der Theorie) | | | | |
| Gehalt (GC): | 99,7 Flächen-% | | | | |
| Reinausbeute: | 87,1 % 1,6-*Bis*(4-cyanophenoxy)hexan | | | | |
| Schmelztemperatur: | 147,2 °C | | | | |
| Elementaranalyse: | berechnet | C, 74,98 % | H, 6,29 %, | N, 8,74 % | S, - |
| | gefunden | C, 74,79 % | H, 6,46 %, | N, 8,59 % | S, 0,02 % |
| ¹H-NMR (CDCl₃, RT): | *δ*[ppm] = 7,55 (d, ³*J* = 9,5 Hz, 4H, Ar-H), 6,91 (t, ³*J* = 7,0 Hz, 4H, Ar-H), 3,99 (t, ³*J*=6,5 Hz, 4H, CH₂), 1,83 (m, 4H, CH₂), 1,53 (m, 4H, CH₂). | | | | |
| ¹³C-NMR (CDCl₃, RT): | *δ*[ppm] = 162,3, 134,0, 119,3, 115,2, 103,8, 68,2, 28,9, 25,8. | | | | |

### Diskussion:

Am Beispiel von 1,6-*Bis*(4-cyanophenoxy)hexan wurde unter streng vergleichenden Bedingungen der besondere Vorteil der Kombination von Dimsyl in einem polaren aprotischen Lösungsmittel herausgearbeitet.

Im Vergleichsbeispiel 1 erfolgt die Umsetzung in reinem DMSO. DMSO dient somit einerseits als Quelle für das in situ generierte Dimsyl-Anion, andererseits fungiert es als polares, aprotisches Lösungsmittel. Unter diesen Bedingungen wird eine sehr hohe, formale Ausbeute an isoliertem Produkt von 91,1 % erhalten. Allerdings lässt die Reinheit des Produkts stark zu wünschen übrig. Der Gehalt an 1,6-*Bis*(4-cyanophenoxy)hexan liegt bei lediglich 86,1 Flächen-% (GC). Somit errechnet sich eine Reinausbeute von 78,4 % 1,6-*Bis*(4-cyanophenoxy)hexan. Auch die Elementaranalyse weicht stark von den theoretischen Werten für 1,6-*Bis*(4-cyanophenoxy)hexan ab. Insbesondere der hohe Schwefelwert von 0,90 % lässt zahlreiche Verunreinigungen vermuten, die aus dem Lösungsmittel DMSO herrühren.

Im Vergleichsbeispiel 2 erfolgt die Umsetzung in reinem NMP ohne jedweden Zusatz von DMSO. Die Umsetzung wird zwar in einem polaren, aprotischen Lösungsmittel geführt. Es fehlt jedoch eine Quelle für das Dimsyl-Anion. Unter diesen Bedingungen wird eine unbefriedigende, formale Ausbeute an isoliertem Produkt von nur 57,4 % erhalten. Die Reinheit ist mit einem Gehalt 99,2 Flächen-% 1,6-*Bis*(4-cyanophenoxy)hexan hoch. Es errechnet sich somit eine Reinausbeute von 56,9 % 1,6*-Bis*(4-cyanophenoxy)hexan. Weitere Untersuchungen zeigten, dass zu diesem Zeitpunkt der Umsatz noch nicht vollständig war (nicht abgebildet). Diese geringe Raum-Zeit-Ausbeute wird durch das Fehlen des katalytisch wirkenden Dimsyl-Anions erklärt.

Im Beispiel 14 erfolgt die Umsetzung in NMP unter Zusatz von einem Äquivalent DMSO pro umzusetzender Hydroxygruppe. DMSO dient somit als Quelle für das in situ generierte Dimsyl-Anion. NMP fungiert es als polares, aprotisches Lösungsmittel. Unter diesen Bedingungen wird sowohl eine sehr hohe, formale Ausbeute an isoliertem Produkt von 87,4 % erhalten. Gleichzeitig wird auch eine hohe Reinheit des Produkts erzielt. Der Gehalt an 1,6-*Bis*(4-cyanophenoxy)hexan liegt bei 99,7 Flächen-% (GC). Somit errechnet sich eine Reinausbeute von 87,1 % 1,6-*Bis*(4-cyanophenoxy)hexan, die signifikant über den Reinausbeuten der Vergleichsbeispiele liegt. Auch die Werte der Elementaranalyse stimmen gut mit den theoretischen Werten für 1,6-*Bis*(4-cyanophenoxy)hexan überein.

Die Vorteile eines Produkts hoher Reinheit, das mit hoher Raum-Zeit-Ausbeute erhalten wird, kann folglich durch Einsatz geeigneter Mengen Dimsyl in Kombination mit einem von DMSO verschiedenen, polaren aprotischen Lösungsmittel in einfacher Weise realisiert werden.

## Patentansprüche

1. Verfahren zur Herstellung von Alkoxybenzonitrilen gemäß Formel (I) 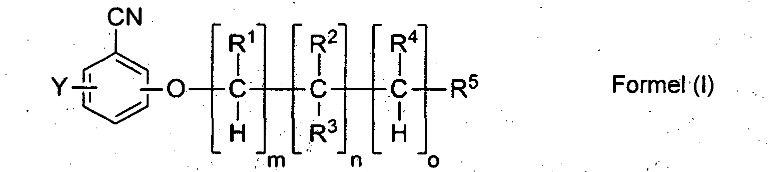 worin für die Reste Y und R5 gleichzeitig oder unabhängig voneinander gilt:
R⁵ = Wasserstoff, Hydroxy oder ein Rest gemäß Formel (II) mit p = eine Zahl von 0 bis 4 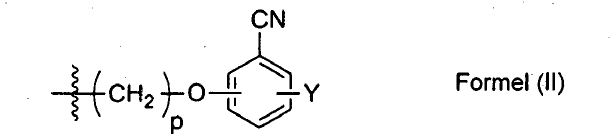
Y = Wasserstoff, Fluor, Chlor, Brom oder Iod;
und worin für die Reste R¹, R², R³, R⁴ und die Indices m, n, o gleichzeitig oder unabhängig voneinander gilt:
a) R¹, R², R³, R⁴ = gleichzeitig oder unabhängig voneinander Wasserstoff, Hydroxy, C1-C6-Alkyl, C1-C6-Hydroxyalkyl oder
ein Rest gemäß oben angegebener Formel (II) und Bedeutung;
m = eine Zahl von 1 bis 4;
n, o = gleichzeitig oder unabhängig voneinander eine Zahl von 0 bis 4;
oder
b) R¹ und R² = gemeinsam unter Bildung eines Ringes C1- bis C6-Alkylen, oder C1-bis C6-Hydcoxyalkylen;
R³, R⁴ = gleichzeitig oder unabhängig voneinander Wasserstoff, Hydroxy, C1-C6-Alkyl, C1-C6-Hydroxyalkyl oder
ein Rest gemäß oben angegebener Formel (II) und Bedeutung;
m, n = gleichzeitig oder unabhängig voneinander eine Zahl von 1 bis 4;
o = eine Zahl von 0 bis 4;
oder
c) R¹ und R⁴ = gemeinsam unter Bildung eines Ringes C1- bis C6-Alkylen, oder C1- bis C6-Hydroxyalkylen;
R², R³ = gleichzeitig oder unabhängig voneinander Wasserstoff, Hydroxy, C1-C6-Alkyl, C1-C6-Hydroxyalkyl oder
ein Rest gemäß oben angegebener Formel (II) und Bedeutung;
m, o = gleichzeitig oder unabhängig voneinander eine Zahl von 1 bis 4;
n = eine Zahl von 0 bis 4;
in dem ein Halogenbenzonitril gemäß Formel (III) mit einem Alkohol gemäß Formel (IV) zur Reaktion gebracht wird 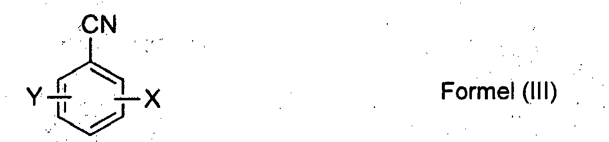 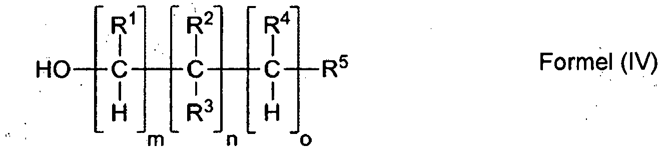 worin die Reste R¹, R², R³, R⁴, R⁵, Y und die Indices m, n, o die oben angegebene Bedeutung aufweisen und für den Rest X gilt:
X = Fluor, Chlor, Brom oder Iod;
**dadurch gekennzeichnet, dass**
die Reaktion in einem von Dimethylsulfoxid verschiedenen, polaren aprotischen Lösungsmittel in Gegenwart von Natrium-methylsulfinylmethid durchgeführt wird, wobei das polare aprotische Lösungsmittel eine Permittivität von über 15 aufweist.

2. Verfahren nach Anspruch 1, **dadurch gekennzeichnet, dass** als polares, aprotisches Lösungsmittel ein Lösungsmittel oder ein Lösungsmittelgemisch verwendet wird, das mindestens ein Lösungsmittel ausgewählt aus der Gruppe N,N-Dimethylformamid, N,N-Dimethylacetamid, N-Methylpyrrolidon, N-Ethylpyrrolidon oder, Sulfolan umfasst.

3. Verfahren nach einem der Ansprüche 1 oder 2, **dadurch gekennzeichnet, dass** das Natrium-methylsulfinylmethid dem Reaktionsgemisch aus Halogenbenzonitril gemäß Formel (III) und Alkohol gemäß Formel (IV) zugegeben wird.

4. Verfahren nach einem der vorhergehenden Ansprüche 1 oder 2, **dadurch gekennzeichnet, dass** das Natrium-methylsulfinylmethid in dem polaren, aprotischen Lösungsmittel in situ hergestellt wird.

5. Verfahren nach mindestens einem der vorhergehenden Ansprüche, **dadurch gekennzeichnet, dass** das Natrium-methylsulfinylmethid in situ in dem Reaktionsgemisch aus Halogenbenzonitril gemäß Formel (III) und Alkohol gemäß Formel (IV) hergestellt wird.

6. Verfahren nach Anspruch 5, **dadurch gekennzeichnet, dass** das Natrium-methylsulfinylmethid in situ durch Zugabe von Natriumhydrid zum Reaktionsgemisch aus Halogenbenzonitril gemäß Formel (III) und Alkohol gemäß Formel (IV) hergestellt wird.

7. Verfahren nach mindestens einem der vorhergehenden Ansprüche, **dadurch gekennzeichnet, dass** das Natrium-methylsulfinylmethid in einem molaren Verhältnis Nätrium-methylsulfinylmethid zu Hydroxygruppen des Alkohols gemäß Formel (IV) im Bereich von 1,5 : 1 bis 1 : 1 eingesetzt wird.

8. Verfahren nach mindestens einem der vorgenannten Ansprüche, **dadurch gekennzeichnet, dass** die Reaktion bei einer Temperatur von 0 bis 150 °C, insbesondere von 20 bis 80 °C erfolgt.

9. Verfahren nach mindestens einem der vorgenannten Ansprüche, **dadurch gekennzeichnet, dass** als Halogenbenzonitril ein Halogenbenzonitril ausgewählt aus der Gruppe p-Chlorbenzonitril oder o-Chlorbenzonitril eingesetzt wird.

10. Verfahren nach mindestens einem der vorgenannten Ansprüche, **dadurch gekennzeichnet, dass** als Alkohol ein Alkohol ausgewählt aus der Gruppe 1-Hexanol, 2,2-Dimethylpropan-1,3-diol, Cyclohexanol, 1,2-Ethandiol, 1,3-Propandiol, 1,4-Butandiol, 1,5-Pentandiol, 1,6-Hexandiol, 1,12-Dodecandiol, 12-(4-Cyanophenoxy)dodecan-1-ol oder Pentaerythrit eingesetzt wird.

## Claims

1. Method for producing alkoxybenzonitriles according to formula (I) 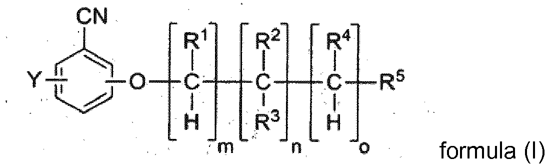 where, for the functional groups Y and R⁵, simultaneously or independently of one another,
R⁵ = hydrogen, hydroxy or
a functional group according to formula (II) where p = a number from 0 to 4 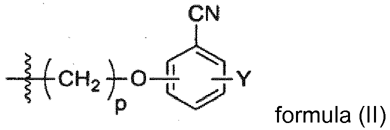
Y = hydrogen, fluorine, chlorine, bromine or iodine;
and where, for the functional groups R¹, R², R³, R⁴ and the values m, n, o, simultaneously or independently of one another:
a) R¹, R², R³, R⁴ = simultaneously or independently of one another hydrogen, hydroxy, C1-C6 alkyl, C1-C6 hydroxyalkyl or
a functional group according to formula (II) and meaning indicated above;
m = a number from 1 to 4;
n, o = simultaneously or independently of one another a number from 0 to 4;
or
b) R¹ and R² = together forming a ring, C1-C6 alkylene or C1-C6 hydroxyalkylene;
R³, R⁴ = simultaneously or independently of one another hydrogen, hydroxy, C1-C6 alkyl, C1-C6 hydroxyalkyl or
a functional group according to formula (II) and meaning indicated above;
m, n = simultaneously or independently of one another a number from 1 to 4;
o = a number from 0 to 4;
or
c) R¹ and R⁴ = together forming a ring, C1-C6 alkylene or C1-C6 hydroxyalkylene;
R², R³ = simultaneously or independently of one another hydrogen, hydroxy, C1-C6 alkyl, C1-C6 hydroxyalkyl or
a functional group according to formula (II) and meaning indicated above;
m, o = simultaneously or independently of one another a number from 1 to 4;
n = a number from 0 to 4;
in which a halogen benzonitrile according to formula (III) is reacted with an alcohol according to formula (IV): 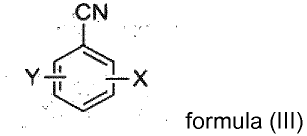 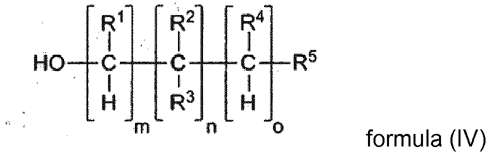 where the functional groups R¹, R², R³, R⁴, R⁵, Y and the values m, n, o have the meaning indicated above, and for the functional group X:
X = fluorine, chlorine, bromine or iodine;
**characterised in that**
the reaction in a polar aprotic solvent that is different from dimethyl sulfoxide is carried out in the presence of sodium methylsulfinylmethylide, the polar aprotic solvent having a permittivity of more than 15.

2. Method according to claim 1, **characterised in that** a solvent or solvent mixture that comprises at least one solvent selected from the group of N,N-dimethylformamide, N,N-dimethylacetamide, N-methylpyrrolidone, N-ethylpyrrolidone or sulfolane is used as the polar aprotic solvent.

3. Method according to either claim 1 or claim 2, **characterised in that** the sodium methylsulfinylmethylide is added to the reaction mixture consisting of halogen benzonitrile according to formula (III) and alcohol according to formula (IV).

4. Method according to either of the preceding claims 1 or 2, **characterised in that** the sodium methylsulfinylmethylide in the polar aprotic solvent is produced in situ.

5. Method according to at least one of the preceding claims, **characterised in that** the sodium methylsulfinylmethylide is produced in situ in the reaction mixture consisting of halogen benzonitrile according to formula (III) and alcohol according to formula (IV).

6. Method according to claim 5, **characterised in that** the sodium methylsulfinylmethylide is produced in situ by adding sodium hydride to the reaction mixture consisting of halogen benzonitrile according to formula (III) and alcohol according to formula (IV).

7. Method according to at least one of the preceding claims, **characterised in that** the sodium methylsulfinylmethylide is used in a molar ratio of sodium methylsulfinylmethylide to hydroxy groups of the alcohol according to formula (IV) in the range of from 1.5:1 to 1:1.

8. Method according to at least one of the preceding claims, **characterised in that** the reaction takes place at a temperature of from 0 to 150°C, in particular of from 20 to 80°C.

9. Method according to at least one of the preceding claims, **characterised in that** a halogen benzonitrile selected from the group of p-chlorobenzonitrile or o-chlorobenzonitrile is used as the halogen benzonitrile.

10. Method according to at least one of the preceding claims, **characterised in that** an alcohol selected from the group of 1-hexanol, 2,2-dimethylpropane-1,3-diol, cyclohexanol, 1,2-ethanediol, 1,3-propanediol, 1,4-butanediol, 1,5-pentanediol, 1,6-hexanediol, 1,12-dodecanediol, 12-(4-cyanophenoxy)dodecan-1-ol or pentaerythritol is used as the alcohol.

## Revendications

1. Procédé de production d'alcoxybenzonitriles selon la formule (I) 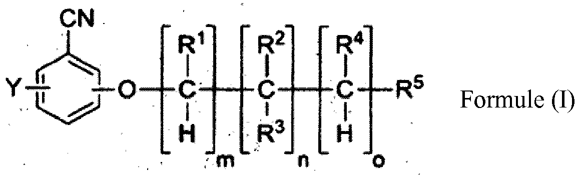 où pour les groupements Y et R⁵ on a simultanément ou indépendamment l'un de l'autre:
R⁵ = hydrogène, hydroxy ou
un groupement selon la formule (II) avec p = un nombre de 0 à 4 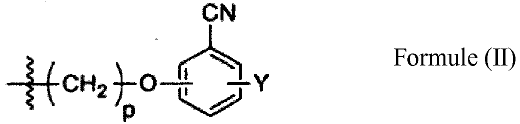
Y = hydrogène, fluor, chlore, brome ou iode;
et où pour les groupements R¹, R², R³, R⁴ et les indices m, n, o on a simultanément ou indépendamment les uns des autres:
(a) R¹, R², R³, R⁴ = simultanément ou indépendamment les uns des autres hydrogène, hydroxy, C1-C6-alkyle, C1-C6-hydroxyalkyle ou
un groupement selon la formule (II) et la signification indiquées ci-dessus;
m = un nombre de 1 à 4;
n, o = simultanément ou indépendamment l'un de l'autre un nombre de 0 à 4;
ou
b) R¹ et R² = ensemble avec formation d'un cycle C1 à C6-alkylène ou C1 à C6-hydroxyalkylène;
R³, R⁴ = simultanément ou indépendamment les uns des autres hydrogène, hydroxy, C1-C6-alkyle, C1-C6-hydroxyalkyle ou
un groupement selon la formule (II) et la signification indiquées ci-dessus;
m, n = simultanément ou indépendamment l'un de l'autre un nombre de 1 à 4;
o = un nombre de 0 à 4;
ou
c) R¹ et R⁴ = ensemble avec formation d'un cycle C1 à C6-alkylène ou C1 à C6-hydroxyalkylène;
R², R³ = simultanément ou indépendamment les uns des autres hydrogène, hydroxy, C1-C6-alkyle, C1-C6-hydroxyalkyle ou
un groupement selon la formule (II) et la signification indiquées ci-dessus;
m, o = simultanément ou indépendamment l'un de l'autre un nombre de 1 à 4;
n = un nombre de 0 à 4;
dans lequel un halogénobenzonitrile selon la formule (III) est mis à réagir avec un alcool selon la formule (IV) 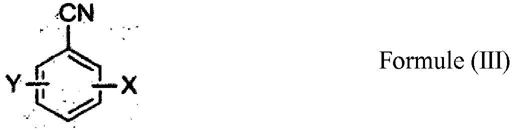 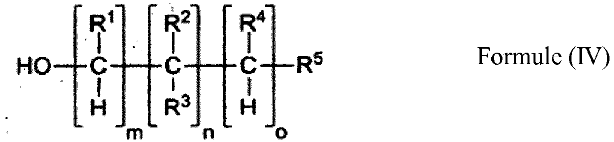 où les groupements R¹, R², R³, R⁴, R⁵, Y et les indices m, n, o présentent la signification indiquée ci-dessus et pour le groupement X on a:
X = fluor, chlore, brome ou iode;
**caractérisé en ce que**
la réaction est conduite dans un solvant aprotique polaire différent du diméthylsulfoxyde en présence de méthylsulfinylméthylure de sodium, où le solvant aprotique polaire présente une permittivité supérieure à 15.

2. Procédé selon la revendication 1, **caractérisé en ce que** l'on utilise comme solvant aprotique polaire un solvant ou un mélange de solvants qui comprend au moins un solvant choisi dans le groupe N,N-diméthylformamide, N,N-diméthylacétamide, N-méthylpyrrolidone, N-éthylpyrrolidone ou sulfolane.

3. Procédé selon l'une des revendications 1 ou 2, **caractérisé en ce que** le méthylsulfinylméthylure de sodium est ajouté au mélange réactionnel d'halogénobenzonitrile selon la formule (III) et d'alcool selon la formule (IV).

4. Procédé selon l'une des revendications 1 ou 2 précédentes, **caractérisé en ce que** le méthylsulfinylméthylure de sodium est produit in situ dans le solvant aprotique polaire.

5. Procédé selon au moins l'une des revendications précédentes, **caractérisé en ce que** le méthylsulfinylméthylure de sodium est produit in situ dans le mélange réactionnel d'halogénobenzonitrile selon la formule (III) et d'alcool selon la formule (IV).

6. Procédé selon la revendication 5, **caractérisé en ce que** le méthylsulfinylméthylure de sodium est produit in situ par addition d'hydrure de sodium au mélange réactionnel d'halogénobenzonitrile selon la formule (III) et d'alcool selon la formule (IV).

7. Procédé selon au moins l'une des revendications précédentes, **caractérisé en ce que** le méthylsulfinylméthylure de sodium est utilisé dans un rapport molaire méthylsulfinylméthylure de sodium à groupes hydroxy de l'alcool selon la formule (IV) dans le domaine de 1,5 : 1 à 1 : 1.

8. Procédé selon au moins l'une des revendications précédentes, **caractérisé en ce que** la réaction a lieu à une température de 0 à 150°C, en particulier de 20 à 80°C.

9. Procédé selon au moins l'une des revendications précédentes, **caractérisé en ce que** l'on utilise comme halogénobenzonitrile un halogénobenzonitrile choisi dans le groupe p-chlorobenzonitrile ou o-chlorobenzonitrile.

10. Procédé selon au moins l'une des revendications précédentes, **caractérisé en ce que** l'on utilise comme alcool un alcool choisi dans le groupe 1-hexanol, 2,2-diméthylpropane-1,3-diol, cyclohexanol, 1,2-éthanediol, 1,3-propanediol, 1,4-butanediol, 1,5-pentanediol, 1,6-hexanediol, 1,12-dodécanediol, 12-(4-cyanophénoxy)dodécan-1-ol ou pentaérythritol.
